# EUROPÄISCHE PATENTSCHRIFT

(11) **EP 1 320 779 B1**
(45) Veröffentlichungstag und Bekanntmachung des Hinweises auf die Patenterteilung: **08.12.2004**
(21) Anmeldenummer: 01982234.5
(22) Anmeldetag: 01.09.2001
(51) Int. Cl.: G02B 21/22, G02B 21/00, G02B 17/04

(54) **BILDUMKEHRSYSTEM, OPHTHALMOSKOPIE-VORSATZMODUL UND OPERATIONSMIKROSKOP**
IMAGE REVERSION SYSTEM, ADDITIONAL OPHTHALMOSCOPY MODULE AND OPERATIONAL MICROSCOPE
SYSTEME DE REDRESSEMENT DE L'IMAGE, MODULE D'OPHTALMOSCOPIE ADDITIONNEL ET MICROSCOPE D'OPERATION

(30) Priorität: 26.09.2000 DE 10047617; 17.08.2001 DE 10140402
(43) Veröffentlichungstag der Anmeldung: 25.06.2003
(60) Teilanmeldung: 03004705.4
(73) Patentinhaber: Carl Zeiss, 89518 Heidenheim (Brenz) (DE)
(72) Erfinder: GOTTWALDT, Klaus, D-73447 Oberkochen (DE); MERZ, Franz, D-73433 Aalen (DE); REIMER, Peter, D-73479 Ellwangen (DE); STRÄHLE, Fritz, D-73540 Heubach (DE)
(86) Internationale Anmeldenummer: PCT/EP2001/010094
(87) Internationale Veröffentlichungsnummer: WO 2002/027379

(56) Entgegenhaltungen:
- WO-A-91/15150
- ES-A- 2 116 933
- US-A- 2 625 853
- US-A- 4 597 644
- US-A- 5 200 773
- US-A- 5 227 914
- US-A- 5 321 447
- US-A- 5 535 060
- US-A- 6 081 371
- PATENT ABSTRACTS OF JAPAN vol. 1999, no. 08, 30. Juni 1999 (1999-06-30) -& JP 11 084263 A (OLYMPUS OPTICAL CO LTD), 26. März 1999 (1999-03-26)

## Beschreibung

Die Erfindung betrifft System zur Bildumkehr mit einem ersten Spiegel zur Umlenkung eines Strahlenbündels aus einer Einfallsrichtung in eine erste Richtung, die quer zu der Einfallsrichtung ist, einem zweiten Spiegel zur Umlenkung des an dem ersten Spiegel umgelenkten Strahlenbündels in eine zweite Richtung, die quer zu der ersten Richtung ist, einem dritten Spiegel zur Umlenkung des an dem zweiten Spiegel umgelenkten Strahlenbündels in eine dritte Richtung, die quer zu der zweiten Richtung ist, und einem vierten Spiegel zur Umlenkung des an dem dritten Spiegel umgelenkten Strahlenbündels in eine Richtung, die quer zu der dritten Richtung ist, wobei die ersten, zweiten, dritten und vierten Spiegel jeweils Flächennormalen haben, welche in einem Winkel zueinander stehen, und wenigstens zwei Spiegel von einer Oberfläche eines 90°-Prismas gebildet werden. Unter Flächennormalen, die zueinander in einem Winkel stehen, sind dabei Flächennormalen zu verstehen, die einen Winkel einschließen, der von 0° und 180° verschieden ist. Dies bedeutet, daß diese Flächennormalen zueinander weder parallel noch antiparallel sind. Die Erfindung betrifft weiter ein mit einem solchen System zur Bildumkehr ausgestattetes Operationsmikroskop-Vorsatzmodul und und ein mit einem solchen Vorsatzmodul ausgerüstetes Mikroskop, insbesondere Operationsmikroskop.

Ein System zur Bildumkehr der eingangs genannten Art ist aus der DE 38 26 069 C2 bekannt. Dort wird vorgeschlagen, für ein binokulares Operationsmikroskop zur Bildumkehr und seitlichen Vertauschung zweier Beobachtungsstahlengänge ein Prismensystem vorzusehen, das aus acht 90°-Prismen aufgebaut ist. Bei jedem dieser 90°-Prismen wirkt einem dem 90°-Winkel gegenüberliegende Prismenfläche als Spiegel. Ein jeder Beobachtungsstrahlengang wird mittels eines solchen 90°-Prismas in eine zur optischen Achse des Mikroskop-Linsensystems senkrechte Ebene ausgekoppelt, um nach zweifacher Reflexion bildverkehrt dem jeweils anderen Beobachtungsstrahlengang zugeführt zu werden. Dieses System zur Bildumkehr ist im Mikroskop zwischen dem Mikroskoptubus und einem Vergrößerungswechsler angeordnet. Es kann in den Beobachtungsstrahlengang des Operationsmikroskops ein- und ausgeschaltet werden.

In "Neumann-Schröder: Bauelemente der Optik, Hanser Verlag München, 1992" ist auf Seite 174 beschrieben, wie durch Mehrfachreflexion an den Oberflächen von Porro-Prismen und von verkürzten Porro-Prismen ein bildumkehrender Strahlengang bereitgestellt werden kann.

Die DE 200 21 955 U1 offenbart ein Operationsmikroskop mit Vorsatzmodul, das für die Durchführung von Operationen im hinteren Augenabschnitt mit einer Ophthalmoskopierlupe ausgelegt ist. Dieses Vorsatzmodul umfaßt ein auf einer Prismenkonstruktion basierendes System zur Bildumkehr.

Es wird unterhalb des Operationsmikroskop-Hauptobjektivs angeordnet und ermöglicht einem Betrachter eine seitenrichtige Darstellung des Augenhintergrunds.

Aus der DE 41 14 646 A1 ist ein Operationsmikroskop mit Ophthalmoskopie-Vorsatzmodul bekannt, das unterhalb des Mikroskops-Hauptobjektivs in Verlängerung des Mikroskoptubus angeordnet ist. Dieses Ophthalmoskopie-Vorsatzmodul hat eine oder mehrere zum Objektiv weisende Ophthalmoskopierlinsen, die dazu dienen, in einer ersten Zwischenbildebene ein höhen- und seitenverkehrtes Bild des Augenhintergrunds eines Patienten zu erzeugen. Über ein optisches System zur Bildaufrichtung und Pupillenvertauschung wird das Bild dieser ersten Zwischenbildebene aufgerichtet und seitenrichtig in eine zweite Zwischenbildebene abgebildet. Das Bild dieser zweiten Zwischenbildebene kann ein Mikroskop-Beobachter durch das Mikroskop-Hauptobjektiv und eine zum Mikroskop-Hauptobjektiv weisende verschiebbare Linse sehen. Mittels des Vorsatzmoduls vermag er so den interessierenden Abschnitt eines Patientenauges zu fokussieren.

Bei Verwendung eines solchen Ophthalmoskopie-Vorsatzmoduls an einem Operationsmikroskop steht einem Operateur allerdings nur ein relativ geringer Arbeitsraum zur Verfügung. Dies liegt daran, daß zur Abbildung des Augenhintergrundes eines Patientenauges das Ophthalmoskopier-Linsensystem knapp oberhalb der Augenhornhaut angeordnet werden muß. Für Kontaktgläser, mit denen entsprechend einer Ophthalmoskopierlupe der Augenhintergrund sichtbar gemacht werden kann und bei denen ein vergleichsweise großer Arbeitsraum für den Operateur möglich ist, sind solche Ophthalmoskopie-Vorsatzmodule nicht ausgelegt.

Das in der DE 41 14 646 A1 beschriebene Ophthalmoskopie-Vorsatzmodul ist auch nicht für eine gleichwertige Operationsgebiet-Mitbeobachtung durch einen Operationsassistenten ausgelegt. Um eine Operationsgebiet-Mitbeobachtung zu ermöglichen, muß das verwendete Operationsmikroskop mit Strahlauskoppeleinheiten im Bereich des Mikroskoptubus versehen werden, die einen Beobachtungsstrahlengang in jeweils zwei Teilstrahlen für Hauptbeobachter und Mitbeobachter aufteilen. Diese Bauweise gewährleistet zwar, daß Haupt- und Mitbeobachter grundsätzlich das gleiche Bild sehen, sie hat jedoch einen deutlichen Helligkeitsverlust des von Haupt- und Mitbeobachter wahrgenommen Bildes zur Folge.

Ein weiters stereoskopisches Operationsmikroskop, bei dem für Augenoperationen zur Abbildung des Augenhintergrundes eines Patientenauges Ophthalmoskopierlupen bzw. Kontaktgläser eingesetzt werden können, ist in der DE 299 05 969 U1 beschrieben. Dieses Operationsmikroskop umfaßt eine in den Strahlengang vor dem Mikroskop-Hauptobjektiv einschwenkbare Zusatzlinse. Oberhalb des Vergrößerungswechslers befindet sich im Mikroskoptubus ein ein- und ausschiebbares System zur Bildumkehr, das es ermöglicht, für einen Operateur ein seiten- und pupillenrichtiges Abbild des Augenhintergrunds zu generieren. Diese Anordnung des Systems zur Bildumkehr hat jedoch einen relativ hohen Mikroskopaufbau zur Folge. Dies führt zu einer entsprechend hohen Einblickhöhe und ein Operateur kann dann nur eine ungünstige Arbeitshaltung einnehmen. Außerdem wird bei diesem Konstruktionsprinzip das für einen Beobachter zu sehende Mikroskopbild nicht durch die Größe des Mikroskop-Hauptobjektivs, sondern durch die Dimensionierung des Systems zur Bildumkehr begrenzt. Dies hat zur Folge, daß ein Beobachter ein vignettiertes Mikroskopbild wahrnimmt. Wird ferner das Mikroskop einerseits zur Untersuchung des Augenhintergrunds eines Patientenauges zusammen mit Ophthalmoskopierlupe bzw. Kontaktglas eingesetzt und muß dann zur Betrachtung der Augenhornhaut die Ophthalmoskopierlupe bzw. das Kontaktglas aus dem Strahlengang entfernt werden, so ist es erforderlich, mit wechselnden Scharfeinstellungen des Mikroskops zu arbeiten. Zum einen behindert dies den Operationsablauf, zum anderen wird hierdurch auch die Brennebene des optischen Systems verlagert. Letzteres hat für, den Beobachter einen unerwünschten Vergrößerungswechsel zur Folge. Außerdem ist es bei diesem Konstruktionsprinzip nicht möglich, unerwünschte Abbildungsfehler von Zusatzlinse, Ophthalmoskopierlupen bzw. Kontaktgläsern zu korrigieren.

Aus der DE 35 39 009 A1 ist ein Vorsatzmodul für ein stereoskopisches Operationsmikroskop bekannt, das ein vor dem Mirkroskop-Hauptobjektiv angeordnetes System zur Bildumkehr umfaßt und eine Ophthalmoskopierlinse aufweist. Mittels dieser Ophthalmoskopierlinse wird der Augenhintergrundes in eine Zwischenbildebene abgebildet, die sich im Vorsatzmodul befindet. Das Bild dieser Zwischenbildebene wird über eine Feldlinse und das System zur Bildumkehr in das Mikroskop-Hauptobjektiv geworfen. Wiederum bedingt ein solcher Mikroskopaufbau einen lediglich geringen Arbeitsraum für einen Operateur und ermöglicht nicht einen Einsatz von Kontaktgläsern, die auf dem Patientenauge angeordnet werden. Soll außerdem während einer Operation abwechselnd der Augenhintergrund oder die Netzhaut eines Patientenauges betrachtet werden, so muß das Vorsatzmodul aus dem Strahlengang entfernt und die Fokussierungseinstellung des Mikroskop-Hauptobjektivs geändert werden.

Aufgabe der Erfindung ist es, ein System zur Bildumkehr mit geringer Bauhöhe zu schaffen, das in einem zur Durchführung von Operationen im hinteren Augenabschnitt ausgelegten Vorsatzmodul für Operationsmikroskope eingesetzt werden kann, sowie ein Vorsatzmodul für ein Operationsmikroskop und ein Mikroskop bereitzustellen, das eine jeweils seitenrichtige Betrachtung von Netzhaut und Augenhintergrund bei größtmöglichem Arbeitsraum für einen Operateur ermöglicht.

Diese Aufgabe wird durch ein System zur Bildumkehr nach Anspruch 1 gelöst, bei dem wenigstens ein Spiegel eine Oberfläche eines Porro-Prismas ist. Bei einem solchen erfindungsgemäßen System zur Bildumkehr ist die als Spiegel wirkende Oberfläche des Porro-Prismas nicht gleichzeitig eine Oberfläche eines 90°-Prismas. Auf diese Weise wird eine kompakte Bauform des Systems zur Bildumkehr ermöglicht.

In Weiterbildung der Erfindung ist das Porro-Prisma als verkürztes Porro-Prisma zweiter Art ausgebildet. Es hat sich herausgestellt, daß ein Porro-Prismensystem zweiter Art eine optische Systemkomponente ist, die sich in hervorragender Weise dazu eignet, einen gewünschten Versatz zwischen einem eintretenden und einem austretenden Strahlenbündel hervorgerufen, wie dies für eine Strahlvertauschung erforderlich ist. Gleichzeitig wird mit dem Porro-Prismensystem zweiter Art eine Bildumkehr bewirkt. Ein Porro-Prisrnensystem zweiter Art ist vergleichsweise kompakt aufgebaut. Die Verwendung eines solchen Prismensystems in Baugruppen für Operationsmikroskope ermöglicht es, die Abmessungen von Operationsmikroskopen hinsichtlich eines größtmöglichen Arbeitsraumes oder einer unerwünschten Verdeckung von Teilen des Operationsfeldes zu optimieren. Dabei wird die Bildumkehr und die Strahlvertauschung mit optischen Komponenten erreicht, die zum gesamten Lichtweg der Strahlbündel mit nur einer vergleichsweise kurzen optischen Weg, länge beitragen.

Indem das System zur Bildumkehr wenigstens einen Spiegel aufweist, der um eine quer zu der Flächennormalen des Spiegels verlaufende Achse drehbar ist, d.h. drehbeweglich an einer Achse festgelegt ist, die zur Flächennormalen des Spiegels nicht parallel ist, wird ein justierbares System zur Bildumkehr geschaffen, das sich zum Einsatz in einem konvergenten Strahlengang eignet.

In Weiterbildung der Erfindung umfaßt das System zur Bildumkehr wenigstens einen Spiegel, der in quer zur Spiegelfläche verlaufender Richtung verschiebbar ist. Unter einer zu einer Spiegelfläche queren Richtung wird dabei jede Richtung verstanden, die nicht parallel zu dieser Spiegelfläche ist. Auf diese Weise wird ein System zur Bildumkehr mit einstellbarem Strahlversatz geschaffen.

In Weiterbildung der Erfindung umfaßt das System zur Bildumkehr wenigstens einen Spiegel, der als Mantelfläche eines 90°-Prismas ausgebildet ist, wobei das 90°-Prisma um eine zu der Mantelfläche des Prismas in etwa parallele Achse drehbar ist. Unter einer zu einer Fläche in etwa parallelen Achse soll dabei jede Achse verstanden werden, die nicht senkrecht zu dieser Fläche steht. Auf diese Weise wird ein einfacher Aufbau des Systems zur Bildumkehr ermöglicht.

In Weiterbildung der Erfindung umfaßt das System zur Bildumkehr wenigstens zwei drehbare und wenigstens zwei verschiebbare Spiegel. Auf diese Weise ist es möglich, ein das System zur Bildumkehr durchlaufendes Strahlenbündel bezüglich der optischen Achse eines Abbildungssystems auszurichten.

In Weiterbildung der Erfindung umfaßt das System zur Bildumkehr zwei zu einer symmetrischen Anordnung ineinandergefügte Porro-Prismensysteme. Auf diese Weise kann die Bauhöhe des Systems minimiert werden.

In Weiterbildung der Erfindung umfaßt das System zur Bildumkehr ein Vorsatzmodul für Operationsmikroskope, das als System zur Bildumkehr ein oder zwei Porro-Prismen zweiter Art umfaßt; es läßt sich besonders kompakt aufbauen.

Mit einem das erfindungsgemäße System zur Bildumkehr enthaltenden Vorsatzmodul läßt sich daher eine hohe Bildqualität bei seitengerichtetem Mikroskopbild erzielen. Indem im Strahlengang eines Operationsmikroskops vor dem Mikroskop-Hauptobjektiv ein Vorsatzmodul mit genau justierbarem System zur Bildumkehr angeordnet wird, kann errechnet werden, daß Grenzwerte für Binokularfehler im Auge eines Beobachters nicht überschritten werden.

Mit einem Fokus-Optiksystem im Vorsatzmodul, das auf der zum Objekt weisenden Seite des Systems zur Bildumkehr angeordnet ist, ist bei Augenoperationen eine wahlweise Verwendung von Ophthalmoskopierlupen und Kontaktgläsern möglich. Indem das Fokus-Optiksystem des Vorsatzmoduls einstellbar gehalten ist, ermöglicht das Vorsatzmodul selbst einen Refraktionsausgleich eines Patientenauges und kann außerdem leicht an unterschiedliche Ophthalmoskopierlupen bzw. Kontaktgläser und das Mikroskop-Hauptobjektiv angepaßt werden. Vorzugsweise hat das Fokus-Optiksystem wenigstens eine Sammellinse. Auf diese Weise ist es möglich, die Fokusebene des Mikroskop-Hauptobjektivs, an die das Vorsatzmodul angeschlossen ist, zu verlagern. Indem das Fokus-Optiksystem im Vorsatzmodul wenigstens eine Streulinse aufweist, wird ein in sich korrigiertes Fokus-Optik-system geschaffen. Außerdem können so eine geringe Bauhöhe und kleine Verschiebungswege des Fokus-Optiksystems erreicht werden. Es können ferner Abbildungsfehler korrigiert werden, die auch auf eine Ophthalmoskopierlupe oder ein Kontaktglas zurückgehen. Vorzugsweise ist bei dem Vorsatzmodul die Sammellinse oder die Streulinse entlang der optischen Achse des Fokus-Optiksystems bewegbar gehalten. Bei dieser Bauweise kann die Fokusebene des optischen Systems aus Operationsmikroskop und Vorsatzmodul variiert werden, ohne daß Änderungen von Einstellungen des Operationsmikroskops vorgenommen werden müssen. Wird das System zur Bildumkehr und das Fokus-Optiksystem im Vorsatzmodul zum Ein- und Ausschalten in den Strahlengang ausgelegt, so ist es möglich, zwischen einer Betrachtung der Cornea und einer Betrachtung des Augenhintergrunds eines Patientenauges bequem hin- und her zu schalten.

In Weiterbildung der Erfindung weist das Vorsatzmodul eine Ophthalmoskopierlupe zur Erzeugung eines Zwischenbildes des Augenhintergrundes eines Patientenauges auf. Auf diese Weise wird ein besonders kompaktes Vorsatzmodul geschaffen.

In Weiterbildung der Erfindung sind bei dem Vorsatzmodul die Brennweiten von Fokus-Optiksystem und Ophthalmoskopierlupe aufeinander abgestimmt.

Hierunter ist zu verstehen, daß bei eingeschaltetem Vorsatzmodul die Brennebene von Mikroskop-Hauptobjektiv und Fokus-Optiksystem im Bereich der Ebene des von der Ophthalmoskopierlupe erzeugten Zwischenbildes liegt, bei ausgeschaltetem System zur Bildumkehr dagegen der Fokus des Operationsmikroskops auf der Cornea eines Patientenauges zu liegen kommt ohne daß es hierfür einer Nachfokussierung bedarf. Auf diese Weise wird eine leichte Einstellung des Operationsmikroskop-Vorsatzmoduls für Augenoperationen ermöglicht, bei denen zwischen einer Betrachtung der Augenhornhaut und Augennetzhaut hin- und hergewechselt werden muß.

Indem in dem Vorsatzmodul eine Strahlvertauschung von wenigstens zwei Beobachtungsstrahlengängen vorgesehen ist, wird eine stereoskopisch richtige Abbildung des Augenhintergrunds bei räumlichem Bildeindruck ermöglicht.

Ein Vorsatzmodul, das ein System zur Strahlvertauschung und Bildumkehr von wenigstens vier binokularen Beobachtungsstrahlengängen umfaßt, gestattet eine Operationsgebiet-Mitbeobachtung ohne daß dies zu einem Helligkeitsverlust im Hauptbeobachtungsbild führt.

Ein mit dem erfindungsgemäßen System zur Bildumkehr ausgerüstetes Operationsmikroskop ist für die Durchführung von Operationen am hinteren Augenabschnitt optimiert.

Vorteilhafte Ausführungsformen der Erfindung sind in den Zeichnungen dargestellt und werden nachfolgend beschrieben.

Es zeigen:
- Figur 1:: eine erste Ausführungsform eines Systems zur Bildumkehr;
- Figur 2:: eine zweite Ausführungsform eines Systems zur Bildumkehr;
- Figur 3:: eine dritte Ausführungsform eines Systems zur Bildumkehr;
- Figur 4:: eine vierte Ausführungsform eines Systems zur Bildumkehr;
- Figur 5a:: eine erste Ausführungsform eines Systems zur Strahlvertauschung und Bildumkehr für einen binokularen Hauptbeobachtungs- und Mitbeobachtungsstrahlengang;
- Figur 5b:: die erste Ausführungsform des Systems zur Strahlvertauschung und Bildumkehr in Frontansicht;
- Figur 6:: eine zweite Ausführungsform eines Systems zur Strahlvertauschung und Bildumkehr für einen binokularen Hauptbeobachtungs- und Mitbeobachtungsstrahlengang;
- Figur 7:: ein Operationsmikroskop mit Vorsatzmodul;
- Figur 8:: einen Schnitt des Strahlengangs durch ein Operationsmikroskop-Hauptobjektiv mit Vorsatzmodul, bei dem ein System zur Strahlvertauschung und Bildumkehr in den Strahlengang geschaltet ist; und
- Figur 9:: einen Schnitt des Strahlengangs durch ein Operationsmikroskop-Hauptobjektiv mit Vorsatzmodul, bei dem ein System zur Strahlvertauschung und Bildumkehr aus dem Strahlengang geschaltet ist.

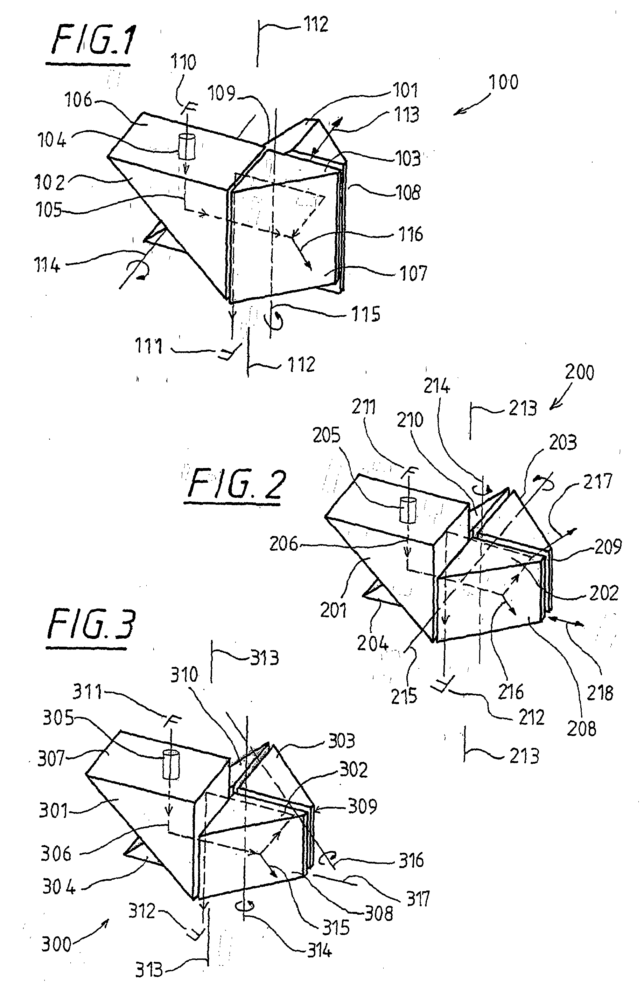

Das System zur Bildumkehr 100 aus Figur 1 besteht aus einem Teilprisma 101, dem zwei 90°-Prismen 102 und 103 zugeordnet sind. Als Spezialfall eines Porro-Prismas ist das Teilprisma 101 als halbes, verkürztes Porro-Prisma zweiter Art ausgebildet. Ein einfallendes Strahlenbündel 104 mit einem Strahlengang 105, das durch die Seitenfläche 106 von Teilprisma 102 tritt, wird an der dem 90°-Winkel gegenüberliegenden als Spiegel wirkenden Seitenfläche reflektiert und aus seiner Einfallsrichtung hin zu dem 90°-Prisma 103 in eine Richtung umgelenkt, die quer zur Einfallrichtung ist. Dort führt die Totalreflexion an der als Spiegel wirkenden Seitenfläche 107 zur Umlenkung des Strahlengangs in das Teilprisma 101. Nach Reflexion an den als Spiegel wirkenden Seitenflächen 108 und 109 von Teilprisma 101 tritt der Mitbeobachtungsstrahlengang seitlich versetzt durch die Grundfläche von Teilprisma 101.

Die Flächennormalen derjenigen Seiten des Teilprismas 101 und der beiden 90°-Prismen 102 und 103, an denen der Strahlengang 105 umgelenkt wird, stehen dabei in einem Winkel zueinander: In einem Winkel von ca. 120° stehen zueinander die Flächennormalen derjenigen Seiten des Teilprismas 101, an denen der Strahlengang 105 umgelenkt wird. Die Flächennormalen der beiden 90°-Prismen 102 und 103, an denen ein Umlenken des Strahlengangs 105 erfolgt, schließen ebenfalls einen Winkel von ca. 120° ein, wogegen die Flächennormalen der Seitenflächen 107 und 108 einen Winkel bilden, der im Bereich von 90° liegt.

Aufgrund der Reflexionen an den Seitenflächen des Systems zur Bildumkehr 100 bewirkt ein das Bild 110 abbildender Strahlengang, der das System zur Bildumkehr 100 durchläuft, ein umgekehrtes Bild 111.

Um beim Einsatz des Systems im Beobachtungsstrahlengang eines Stereomikroskops eine Fokus- und Parallaxe-Differenz zwischen Beobachtungsstrahlengängen zu vermeiden, die durch verschiedene Systeme zur Bildumkehr geführt werden, ist einerseits die Lage der Brennebenen bei der Abbildung durch die verschiedenen Systeme und andererseits die Orientierung von Eintritts- und Austrittsstrahlengang sowie deren Versatz relativ zu einer optischen Achse 112 einstellbar gehalten. Hierzu sind das Teilprisma 101 und die beiden 90°-Prismen 102 und 103 in einem in der Figur nicht dargestellten Halterahmen mit Justiervorrichtungen festgehalten. Diese Justiervorrichtungen ermöglichen es einerseits, das Teilprisma 101 in Richtung einer Achse 113 quer zur als Spiegel wirkenden Seitenfläche 108 hin und her zu verschieben, um so den Abstand dieses Teilprismas 101 zu den 90°-Prismen 102 und 103 einstellen zu können. Andererseits ist in dem Halterahmen das 90°-Prisma 102 zur Justierung um eine Drehachse 114 drehbeweglich festgelegt, die zu der dem 90°-Winkel gegenüberliegenden, als Spiegel wirkenden Seitenfläche von 90°-Prisma 102 parallel ist. Das 90°-Prisma 103 kann um eine Drehachse 114 bewegt werden, die quer zur Flächennormalen der als Spiegel wirkenden Seitenfläche 107 von 90°-Prisma 103 verläuft.

Die Figur 2 zeigt ein System zur Bildumkehr 200 mit vier 90°-Prismen 201, 202, 203 und 204. Ein Strahlenbündel 205 mit einem Strahlengang 206, das durch die Seitenfläche 207 von 90°-Prisma 201 tritt, wird an der dem 90°-Winkel gegenüberliegenden Seitenfläche reflektiert und in einer Richtung zu dem 90°-Prisma 202 umgelenkt, die quer zu seiner Einfallsrichtung ist. Dort führt die Totalreflexion an der Seitenfläche 208 zur Umlenkung des Strahlengangs in querer Richtung in das 90°-Prisma 203. Nach Reflexion an dessen Seitenfläche 209 gelangt der Strahlengang in 90°-Prisma 204, um dann nach neuerlicher Reflexion an der Seitenfläche 210 seitlich versetzt das 90°-Prisma 204 zu verlassen. Entsprechend dem System zur Bildumkehr 100 aus Figur 1 stehen wiederum die Flächennormalen derjenigen Seiten der 90°-Prismen 201, 202, 203 und 204, an denen der Strahlengang 206 umgelenkt wird, in einem Winkel zueinander.

Durch die Reflexionen an den Seitenflächen des Systems zur Bildumkehr 200 führt ein das Bild 211 abbildender Strahlengang, der das System zur Bildumkehr 200 durchläuft, zu einem umgekehrten Bild 212.

Um beim Einsatz dieses Systems im Beobachtungsstrahlengang eines Stereomikroskops wiederum Fokus- und Parallaxe-Differenzen zwischen mehreren Beobachtungsstrahlengängen, die durch verschiedene Systeme zur Bildumkehr geführt werden, zu vermeiden, ist einerseits die Lage der Brennebenen bei der Abbildung durch die verschiedenen Systeme zur Bildumkehr und andererseits die Orientierung von Eintritts- und Austrittsstrahlengang sowie der Versatz relativ zu einer optischen Achse 213 einstellbar gehalten. Hierzu ist ein in der Figur nicht dargestellter Halterahmen mit Justiervorrichtungen vorgesehen, in den die 90°-Prismen 201, 202, 203 und 204 aufgenommen sind.

In diesem Halterahmen sind die 90°-Prismen 201 und 204 unbeweglich gelagert, wogegen das 90°-Prisma 202 zur Justierung um eine Drehachse 214 bewegt werden kann. Diese Drehachse 214 ist zu den als Spiegel wirkenden Seitenflächen 208 und 209 der beiden 90°-Prismen 202 und 203 parallel. Weiter können in dem Halterahmen diese 90°-Prismen 202 und 203 gemeinsam um eine Kippachse 215 eingestellt werden, die quer zu den Flächennormalen 216 und 217 der Seitenflächen 208 und 209 dieser Prismen verläuft, und zusätzlich entlang einer Achse 218 gemeinsam hin- und hergeschoben werden, die wiederum quer zu den Seitenflächen 208 und 209 der Prismen 202 und 203 ist.

In der Figur 3 ist ein weiteres System zur Bildumkehr 300 gezeigt, das in seinem Aufbau weitestgehend dem System zur Bildumkehr 200 aus Figur 2 entspricht. Dieses System umfaßt wiederum vier 90°-Prismen 302, 302, 303 und 304. Ein Strahlenbündel 305 mit einem Strahlengang 306, das durch die Seitenfläche 307 von 90°-Prisma 301 tritt, wird an der dem 90°-Winkel gegenüberliegenden Seitenfläche reflektiert und in das 90°-Prisma 302 gelenkt. Dort führt die Totalreflexion an der Seitenfläche 308 zur Umlenkung des Strahlengangs 306 in das 90°-Prisma 303. Durch Reflexion an dessen Seitenfläche 309 wird der Strahlengang 306 in das 90°-Prisma 304 geführt, um dann nach neuerlicher Reflexion an der Seitenfläche 310 das 90°-Prisma 304 zu verlassen. Aufgrund der Reflexionen an den Seitenflächen des Systems zur Bildumkehr 300 führt ein das Bild 311 abbildender Strahlengang, der das System zur Bildumkehr 200 durchläuft, zu einem seitlich versetzten und umgekehrten Bild 312.

Die Lage der Brennebene bei der Abbildung durch die verschiedenen Systeme einerseits sowie der vom System zur Bildumkehr 300 hervorgerufene Strahlversatz und die Orientierung von Eintritts- und Austrittsstrahlengang relativ zu einer optischen Achse 313 andererseits sind wiederum einstellbar gehalten. Hierzu ist ein in der Figur nicht dargestellter Halterahmen mit Justiervorrichtungen vorgesehen, in den die 90°-Prismen 301, 302, 303 und 304 aufgenommen sind. In diesem Halterahmen sind die 90°-Prismen 301 und 304 unbeweglich gelagert, wogegen das 90°-Prisma 302 zur Justierung um eine Drehachse 314, die parallel zu der als Spiegel wirkenden Seitenfläche 308 des 90°-Prisma 302 verläuft, bewegt werden und das 90°-Prisma 303 unter Ausführung einer Kippbewegung um eine zur Flächennormalen 315 der Seitenfläche 309 quere Drehachse 316 justiert werden kann. Außerdem können in dem Halterahmen die 90°-Prismen 302 und 303 entsprechend dem System zur Bildumkehr 200 aus Figur 2 gemeinsam entlang einer Achse 317 hin- und hergeschoben werden, die wiederum quer zu den Seitenflächen 308 und 309 der 90°-Prismen 302 und 303 ist.

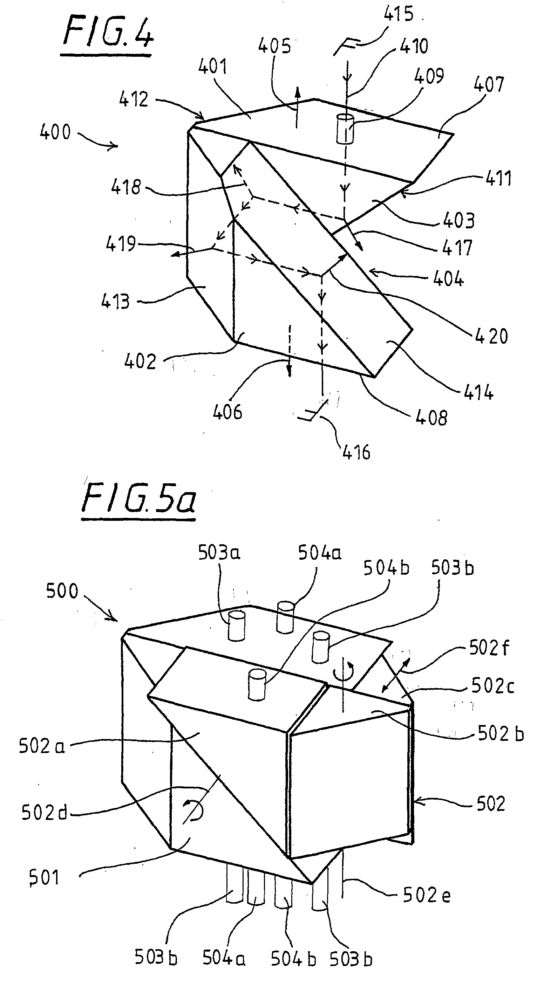

Die Figur 4 zeigt ein System zur Bildumkehr 400 aus Figur 1, das aus zwei identischen Teilprismen 401 und 402 besteht, die jeweils als Spezialfall von Porro-Prismen in Form von halben, verkürzten Porro-Prismen zweiter Art ausgebildet sind. Die Teilprismen 401 und 402 liegen jeweils an der Seite ihrer größten Seitenflächen 403 und 404 so aneinander, daß die nach außen weisenden Flächennormalen 405, 406 der einander entsprechenden Seitenflächen 407 und 408 zueinander parallel stehen.

Ein Strahlenbündel 409 mit einem Strahlengang 410, das durch die Seitenfläche 407 von Teilprisma 401 tritt, wird an der als Spiegel wirkenden Seitenfläche 411 reflektiert und aus seiner Einfallsrichtung in eine Richtung umgelenkt, die quer zur Einfallrichtung ist und in der es auf die Seitenfläche 412 von Teilprisma 401 trifft. Dort führt die Totalreflexion an der als Spiegel wirkenden Seitenfläche 412 zur Umlenkung des Strahlengangs in das Teilprisma 402 in Richtung von dessen Seitenfläche 413. Der Strahlengang wird an der Seitenfläche 413 reflektiert und quer zur Seitenfläche 414 von Teilprisma 402 umgelenkt. Wiederum wird der Strahlengang hier reflektiert um über die Grundfläche von Teilprisma 402 das System zur Bildumkehr 400 zu verlassen. Durch die Reflexionen an den Seitenflächen des Systems zur Bildumkehr 400 bildet ein das Bild 415 abbildender Strahlengang, der das System zur Bildumkehr 400 durchläuft, unter seitlichen Versatz in ein umgekehrtes Bild 416 ab.

Bei dem System zur Bildumkehr 400 stehen die Flächennormalen 417, 418 zueinander in einem Winkel von ca. 120°. Entsprechendes gilt für die Flächennormalen 419 und 420, wogegen die Flächennormalen 418 und 419 mit einem im Bereich von 90° liegenden Winkel zueinander orientiert sind. Die Flächennormalen derjenigen Seiten der Teilprismen 401 und 402, an denen der Strahlengang 410 umgelenkt wird, stehen also wiederum zueinander in einem Winkel.

Die Figur 5a zeigt ein System zur gleichzeitigen Strahlvertauschung und Bildumkehr für einen binokularen Hauptbeobachtungsstrahlengang und einen binokularen Mitbeobachtungsstrahlengang. Das System zur Strahlvertauschung und Bildumkehr 500 ist aus einem ersten System zur Bildumkehr 501 aufgebaut, das demjenigen System zur Bildumkehr 400 aus Figur 4 entspricht, und umfaßt ein System zur Bildumkehr 502 mit nicht weiter dargestellten Halterahmen, das entsprechend dem System zur Bildumkehr 100 aus Figur 1 aufgebaut ist. Dieses erste System zur Bildumkehr 501 und das zweiter System zur Bildumkehr 502 sind zu einer symmetrischen Anordnung zusammengefügt, die auf eine in der Figur 5a nicht weiter dargestellte Halterung montiert ist. An dieser Halterung ist der Halterahmen des Systems zur Bildumkehr 502 festgelegt. Er hält die 90°-Prismen 502a und 502b sowie das als verkürztes Porro-Prisma ausgebildete Teilprisma 502c, wobei das 90°-Prisma 502a zur Justierung um die Drehachse 502d und das 90°-Prisma 502b zur Justierung um die Drehachse 502e bewegt werden können. Weiter ist es in dem Halterahmen möglich, das Teilprisma 502c entlang der Achse 502f zu verschieben.

Für einen binokularen Strahlengang mit Strahlenbündeln 503a und 503b wirkt das System zur Bildumkehr 501 sowohl als System zur Bildumkehr als auch zur Strahlvertauschung. Für das Strahlenbündel 504a eines aus den Strahlenbündeln 504a und 504b bestehenden binokukaren Strahlengangs ruft das System zur Bildumkehr 501 dagegen nur eine Bildumkehr unter gleichzeitigem seitlichen Versatz hervor. Indem das Strahlenbündel 504b des zugehörigen binokularen Strahlengangs über das System zur Bildumkehr 502 geführt wird, wird ein System zur Strahlvertauschung und Bildumkehr 500 von vier Strahlengängen geschaffen.

Aufgrund der Justiermöglichkeit der 90°-Prisma 502a und 502b sowie des Teilprismas 502c im System zur Bildumkehr 502 können Fokus- und Parallaxedifferenzen für den Strahlengang aus den Strahlenbündeln 504a und 504b durch entsprechendes Einstellen der Prismen ausgeglichen werden. Somit kann das System zur Strahlvertauschung und Bildumkehr 500 präzise insbesondere für eine Anordnung in einem konvergenten Strahlengang eingestellt werden, wie dies beispielsweise vor dem Hauptobjektiv eines Operationsmikroskopes der Fall ist.

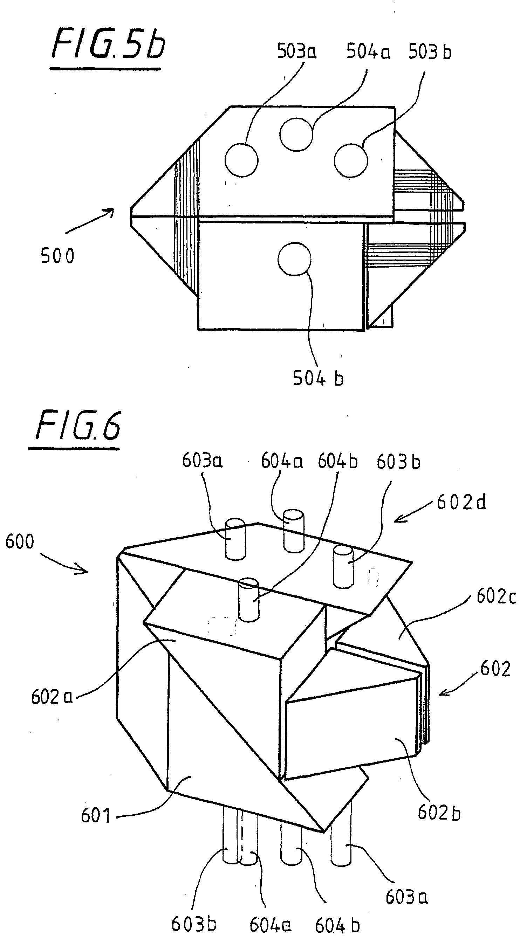

Die Figur 5b zeigt das System zur Strahlvertauschung und Bildumkehr 500 in Frontansicht. Die Beobachtungsstrahlenbündel 503a und 503b sowie 504a und 504b können auf einem sehr engen Raum gehalten werden, der beispielsweise durch den Durchmesser eines Operationsmikroskop-Hauptobjektives bestimmt ist.

In Figur 6 ist eine weitere Ausführungsform 600 für ein System zur gleichzeitigen Strahlvertauschung und Bildumkehr eines binokularen Hauptbeobachtungsstrahlenganges und eines binolularen Mitbeobachtungsstrahlenganges gezeigt. Das System zur Strahlvertauschung und Bildumkehr 600 ist wiederum aus einem ersten System zur Bildumkehr 601 aufgebaut, das demjenigen System zur Bildumkehr 400 aus Figur 4 entspricht, und umfaßt ein System zur Bildumkehr 602 mit nicht weiter dargestelltem Halterahmen, das entsprechend dem System zur Bildumkehr 200 aus Figur 2 oder dem System zur Bildumkehr 300 aus Figur 3 aufgebaut ist. Dieses erste System zur Bildumkehr 601 und das zweiter System zur Bildumkehr 602 sind zu einer symmetrischen Anordnung zusammengefügt. Das System zur Strahlvertauschung und Bildumkehr 600 ist wiederum auf eine in der Figur 6 nicht weiter dargestellte Halterung montiert. An dieser Halterung ist der Halterahmen des Systems zur Bildumkehr 602 angeordnet. Dieser Halterahmen nimmt die vier 90°-Prismen 602a, 602b, 602c und 602d auf, wobei die Lage der 90°-Prismen entsprechend den anhand der Figuren 2 und 3 erläuterten alternativen Konfigurationen für Bewegungsachsen einstellbar ist.

Das System zur Bildumkehr 601 wirkt für einen binokularen Strahlengang mit Strahlenbündeln 603a und 603b als System zur Strahlvertauschung und Bildumkehr. Für das Strahlenbündel 604a eines aus den Strahlenbündeln 604a und 604b bestehenden binokukaren Strahlengangs fungiert es dagegen nur als System zur Bildumkehr, das gleichzeitig einen seitlichen Versatz des Strahlengangs hervorruft. Indem das Strahlenbündel 604b des zugehörigen binokularen Strahlengangs über das System zur Bildumkehr 502 geführt wird, wird ein System zur gleichzeitigen Strahlvertauschung und Bildumkehr 500 von vier Strahlengängen geschaffen.

Aufgrund der Justiermöglichkeit der vier 90°-Prisma die vier 90°-Prismen 602a, 602b, 602c und 602d in dem System zur Bildumkehr 602 ist es wiederum möglich, Fokus- und Parallaxendifferenzen für den Strahlengang aus den Strahlenbündeln 604a und 604b durch entsprechendes Einstellen der Prismen auszugleichen, so daß das System zur Strahlvertauschung und Bildumkehr 600 ebenfalls präzise justiert werden kann, was insbesondere eine Anordnung in einem konvergenten Strahlengang ermöglicht.

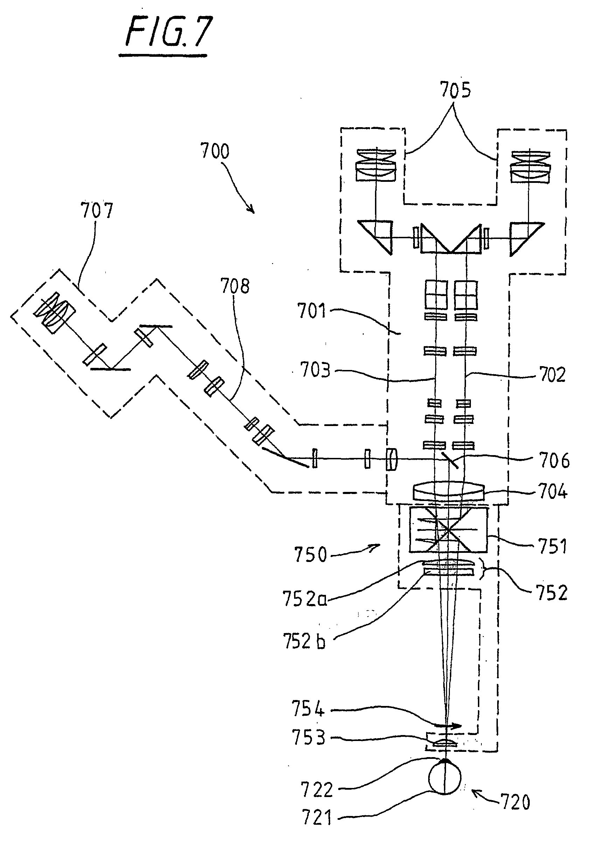

Die Figur 7 zeigt ein als stereoskopisches Operationsmikroskop ausgebildetes Mikroskop 700 mit binokularen Beobachtungsstrahlengängen 702, 703 und 708 für einen Haupt- und Mitbeobachter. Das Mikroskop 700 umfaßt einen Tubus 701, in dem jeweils getrennte Linsensysteme für den linken und rechten Beobachtungsstrahlengang 702, 703 vorgesehen sind. Der linke Beobachtungsstrahlengang 702 und der rechte Beobachtungsstrahlengang 703 treten durch ein gemeinsames Mikroskop-Hauptobjektiv 704 hindurch. Für einen in Fig. 1 nicht weiter dargestellten Hauptbeobachter ist beim Mikroskop 700 eine Okulareinheit 705 vorgesehen. Durch diese Okulareinheit 705 kann ein Operationsgebiet an einem menschlichen Auge 720 betrachtet werden.

Im Tubus 701 des Operationsmikroskopes 700 ist oberhalb des Mikroskop-Hauptobjektives 704 ein Umlenkspiegelsystem 706 vorgesehen, um für einen Mitbeobachter durch eine Okulareinheit 707 und einen binokularen Beobachtungsstrahlengang 708 ebenfalls die Beobachtung des Operationsgebietes zu ermöglichen. Der binokulare Beobachtungsstrahlengang 708 umfaßt wiederum einen linken und rechten Beobachtungsstrahlengang, die zu den linken und rechten Beobachtungsstrahlengängen 702 und 703 durch das Mikroskop-Hauptobjektiv 704 versetzt hindurchtreten. Somit wird eine Mitbeobachtung des Operationsgebietes im Vergleich zum Einsatz einer Strahlteilertechnik ohne Helligkeitsverlust ermöglicht.

Am Tubus 701 des Operationsmikroskopes 700 befindet sich unterhalb des Mikroskop-Hauptobjektivs 704 ein Vorsatzmodul 750 zur seiten- und bildrichtigen Abbildung des Augenhintergrundes 721 des Auges 720. Dieses Vorsatzmodul 750 ist zum schnellen und leichten Wechsel mittels Bajonettverschluß mit dem Tubus 701 verbunden. Das Vorsatzmodul 750 umfaßt ein System zur Bildumkehr und Strahlvertauschung 751, eine Fokus-Optik 752 sowie eine Ophtalmoskopierlupe 753. Das System zur Bildumkehr und Strahlvertauschung 751 im Vorsatzmodul 750 ist für gleichzeitige Bildumkehr und Strahlvertauschung von vier binokularen Beobachtungsstrahlengängen ausgelegt. Es ist im zum Mikroskop-Hauptobjektiv weisenden Bereich des Vorsatzmoduls 750 angeordnet. Die Fokus-Optik 752 befindet sich unterhalb des Systems zur Bildumkehr und Strahlvertauschung 751 in dem zum Operationsgebiet weisenden Bereich des Vorsatzmoduls 750. Sie umfaßt eine Sammellinse 752a und eine Streulinse 752b. Der Fokus-Optik 752 ist eine Ophthalmoskopierlupe 753 zugeordnet.

Zur Durchführung einer Operation an einem menschlichen Auge 720 ist das Mikroskop-Hauptobjektiv 704 des Mikroskops 700 auf dessen Netzhaut 722 scharf gestellt.

Die Fokus-Optik 752 fokussiert die Beobachtungsstrahlengänge 702, 703 und 708 in einer Zwischenbildebene 754, in welche die Ophthalmoskopierlupe 753 ein seitenverkehrtes Bild des Augenhintergrundes 721 wirft. Die Lage dieser Zwischenbildebene 754 wird zum einen durch die Brechkraft der Ophtalmoskopierlupe 753 selbst bestimmt, zum anderen aber auch durch deren Abstand vom Auge, die Augengeometrie selbst und die Brechkraft der Augenlinsen festgelegt. Die Fokus-Optik 752 ist einstellbar ausgeführt, indem die Sammellinse 752a entlang der optischen Achse der Fokus-Optik 722 verschiebbar gelagert ist. Somit kann der Arbeitsabstand des Mikroskops 700 mit Vorsatzmodul 750 von einem menschlichen Auge 120 einstellbar gehalten werden und es ist möglich, Anpassungen an ein ametropes oder aphakes Patientenauge vorzunehmen.

Es ist auch möglich, die Fokus-Optik derart auszuführen, daß die Streulinse verschiebbar ist und die Sammellinse unbeweglich gehalten wird oder beide Linsen entlang der optischen Achse der Fokus-Optik verschoben werden können.

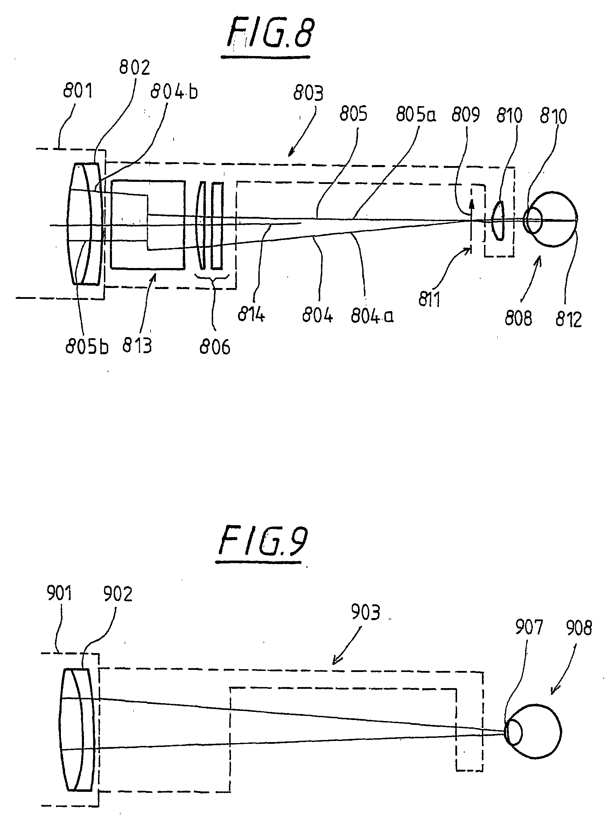

Die Figur 8 zeigt einen Schnitt des unteren Bereiches 801 des Tubus von Mikroskop 700 aus Figur 7 mit einem Verlauf der Beobachtungsstrahlengänge 804, 805 für einen Mitbeobachter.

Die durch das Hauptobjektiv 802 tretenden binokularen Beobachtungsstrahlengänge 804, 805 für die Beobachtungspupillen des Mitbeobachters werden mit der Fokus-Optik 806 bei auf die Netzhaut 807 eines Patientenauges 808 fokussiertem Mikroskop-Hauptobjektiv 802 auf das Zwischenbild 809 der Ophthalmoskopierlupe 810 eingestellt. Die Ophthalmoskopierlupe 810 wirft in eine Zwischenbildebene, 811 ein seitenverkehrtes Bild des Augenhintergrundes 812.

Das System zur Bildumkehr und Strahlvertauschung 813 bewirkt für einen jeden der binokularen Beobachtungsstrahlengänge eine Bildumkehr des seitenverkehrten Bildes von Augenhintergrund 812 in der Zwischenbildebene 811. Von der Zwischenbildebene 811 aus gesehen wird im System zur Bildumkehr und Strahlvertauschung 813 der oberhalb der optischen Achse 814 der Fokus-Optik 806 verlaufende Strahlengang 805a seitenverkehrt in einen unterhalb der optischen Achse 814 der Fokus-Optik 806 verlaufenden Strahlengang 805b gelenkt. Ein unterhalb von der optischen Achse 814 der Fokus-Optik 806 verlaufender Strahlengang 804a wird durch das System zur Bildumkehr und Strahlvertauschung 813 in einen Strahlengang 804b gewandelt, der oberhalb der optischen Achse 814 der Fokus-Optik 806 verläuft. Entsprechend lenkt das System zur Bildumkehr und Strahlvertauschung 813 von der Zwischenbildebene 811 aus gesehen einen in Figur 2 nicht dargestellten binokularen Strahlengang, der links der optischen Achse 814 der Fokus-Optik verläuft, in einen rechts dieser Achse liegenden Strahlengang. Ein Strahlengang rechts von der optischen Achse 814 der Fokus-Optik wird durch das System zur Bildumkehr und Strahlvertauschung 813 in einen links dieser Achse verlaufend Strahlengang umgelenkt. Aufgrund des stereoskopischen Verlauf der Beobachtungsstrahlengänge ergibt sich damit sowohl für Haupt- als auch Mitbeobachter ein seiten- und pupillenrichtiges Bild von Augenhintergrund 812 mit räumlichem Eindruck

Beim Vorsatzmodul 803 sind Ophthalmoskopierlupe 810, Fokus-Optik 806 und das System zur Bildumkehr und Strahlvertauschung 813 mittels eines nicht weiter dargestellten Mechanismus beispielsweise durch Einschieben oder Einschwenken in den optischen Strahlengang ein- und ausschaltbar gehalten.

Figur 9 zeigt einen Schnitt des unteren Bereiches des Tubus 901 von Mikroskop 700 aus Figur 7 mit an das Mikroskop-Hauptobjektiv 902 angeschlossenem Vorsatzmodul 903. Hier sind das System zur Bildumkehr, die Fokus-Optik und die Ophtalmoskopierlupe aus dem Strahlengang geschaltet. In diesem Fall entspricht die Fokus-Ebene der Beobachtungsstrahlengänge derjenigen des Mikroskop-Hauptobjektivs und liegt bei der Augenhornhaut 907 eines untersuchten Patientenauges 908.

Wie bei dem System zur Bildumkehr und Strahlvertauschung umfaßt das Vorsatzmodul 903 zur Schaltung von Ophtalmoskopierlupe und Fokus-Optik 906 in und aus dem Strahlengang jeweils geeignete Schwenk- oder Verschiebemechanismen. Alternativ zu dieser Ausführung ist es auch möglich, einen Mechanismus zur Entfernung des Vorsatzmodules als Ganzes aus dem Strahlengang vorzusehen. Vorteilhafterweise sind solchen Mechanismen steuerbare Antriebe zugeordnet.

Mit dem auf vier Beobachtungsstrahlengänge ausgelegten System zur Strahlvertauschung und Bildumkehr kann ohne Helligkeitsverlust mit einem Mikroskop-Hauptobjektiv eine vollwertige binokulare Mitbeobachtung eines Operationsgebietes bei 0° Blickwinkel und gleichem Stereowinkel wie ein Hauptbeobachter bereitgestellt werden.

Indem Beobachtungsstrahlengänge unabhängig voneinander das Mikroskop-Hauptobjektiv durchdringen, ist die Mitbeobachtung eines Operationsfeldes ohne Helligkeitsverlust für Haupt-und Mitbeobachter im Vergleich zum Einsatz einer Strahlteilertechnik möglich.

Anstatt das System zur Bildumkehr und Strahlvertauschung für eine Bildumkehr und eine Strahlvertauschung von vier binokularen Beobachtungsstrahlengängen auszulegen, ist es auch möglich, das System lediglich für Bildumkehr und Strahlvertauschung von drei Beobachtungsstrahlengängen auszuführen. Etwa können Bildumkehr und Strahlvertauschung für zwei binokulare Hauptbeobachtungsstrahlengänge und einen Mitbeobachtungsstrahlengang vorgesehen werden. Möglich ist auch, lediglich eine Bildumkehr für ein oder zwei Beobachtungsstrahlengänge vorzusehen. In diesem Fall wird jedoch einem Beobachter kein räumliches Mikroskopbild dargeboten. Optional kann ein solches System zur Bildumkehr auch für einen Hauptbeobachtungsstrahlengang und drei Mitbeobachtungsstrahlengänge ausgelegt werden.

Eine modifizierte, in den Figuren nicht dargestellte Ausführungsform des Vorsatzmoduls zum Anbau an ein Mikroskop-Hauptobjektiv ist zur Abbildung des Hintergrundes eines Patientenauges für den Einsatz mit einer externen Ophthalmoskopierlupe bzw. einem Kontaktglas ausgelegt. Ein solches Vorsatzmodul ist demnach ohne Ophthalmoskopierlupe ausgeführt. Jedoch ermöglicht die Fokus-Optik im Vorsatzmodul eine Fokussierung auf das mit Ophthalmoskopierlupe bzw. Kontaktglas erzeugte Zwischenbild des Patientenauges.

## Patentansprüche

1. System zur Bildumkehr (100, 500, 600) mit
- einem ersten Spiegel zur Umlenkung eines Strahlenbündels (104) aus einer Einfallsrichtung in eine erste Richtung, die quer zu der Einfallsrichtung ist, wobei der erste Spiegel als Grundfläche eines ersten 90°-Prismas (102) ausgebildet ist,
- einem zweiten Spiegel (107) zur Umlenkung des an dem ersten Spiegel umgelenkten Strahlenbündels in eine zweite Richtung, die quer zu der ersten Richtung ist, wobei der zweite Spiegel (107) als Grundfläche eines zweiten 90°-Prismas (103) ausgebildet ist,
- einem dritten Spiegel (108) zur Umlenkung des an dem zweiten Spiegel (107) umgelenkten Strahlenbündels in eine dritte Richtung, die quer zu der zweiten Richtung ist, und
- einem vierten Spiegel (109) zur Umlenkung des an dem dritten Spiegel (108) umgelenkten Strahlenbündels in eine Richtung, die quer zu der dritten Richtung ist,
- wobei die ersten, zweiten, dritten und vierten Spiegel jeweils Flächennormalen haben, welche in einem Winkel zueinander stehen,
**dadurch gekennzeichnet, dass**
- der dritte Spiegel (108) und der vierte Spiegel (109) eine Oberfläche eines halben Porro-Prismas (101) bilden
- und das zweite 90°-Prisma (103) um eine quer zu der Flächennormalen (116) seiner Grundfläche verlaufende Achse (115) derart drehbar ist, dass eine Verkippung einer. Fläche des zweiten 90°-Prismas (103) zu einer gegenüberliegenden Fläche des ersten 90°-Prismas (102) eingestellt werden kann.

2. System zur Bildumkehr (200, 300), mit
- einem ersten Spiegel zur Umlenkung eines Strahlenbündels (205) aus einer Einfallsrichtung in eine erste Richtung, die quer zu der Einfallsrichtung ist, wobei der erste Spiegel als Grundfläche eines ersten 90°-Prismas (201, 301) ausgebildet ist,
- einem zweiten Spiegel (208, 308) zur Umlenkung des an dem ersten Spiegel umgelenkten Strahlenbündels in eine zweite Richtung, die quer zu der ersten Richtung ist, wobei der zweite Spiegel (208, 308) als Grundfläche eines zweiten 90°-Prismas (202, 302) ausgebildet ist,
- einem dritten Spiegel (209, 309) zur Umlenkung des an dem zweiten Spiegel (107, 208, 308) umgelenkten Strahlenbündels in eine dritte Richtung, die quer zu der zweiten Richtung ist, und
- einem vierten Spiegel (210, 310) zur Umlenkung des an dem dritten Spiegel (108, 209, 309) umgelenkten Strahlenbündels in eine Richtung, die quer zu der dritten Richtung ist,
- wobei die ersten, zweiten, dritten und vierten Spiegel (107, 208, 308, 108, 209, 309, 109, 210, 310) jeweils Flächennormalen (116, 216, 217, 315) haben, welche in einem Winkel zueinander stehen und
- der dritte Spiegel (209, 309) als Grundfläche eines dritten 90°-Prismas (203, 303) ausgebildet ist und der vierte Spiegel (210, 310) als Grundfläche eines vierten 90°-Prismas (204, 304) ausgebildet ist,
- die zusammengenommen ein halbes Porro-Prisma bilden,
**dadurch gekennzeichnet, dass**
- das zweite 90°-Prisma (203, 303) um eine quer zu der Flächennormalen (216, 315) seiner Grundfläche verlaufende Achse (214,314) derart drehbar ist, dass eine Verkippung einer Fläche des zweiten 90°-Prismas (202, 302) zu einer gegenüberliegenden Fläche des ersten 90°-Prismas (201, 301) eingestellt werden kann.

3. System zur Bildumkehr nach einem der Ansprüche 1 oder 2, **dadurch gekennzeichnet, dass** wenigstens ein Spiegel (108, 208, 209, 308, 309) in quer zur Spiegelfläche verlaufender Richtung (113, 218, 314) verschiebbar ist.

4. System zur Bildumkehr nach einem der Ansprüche 1 bis 3, **dadurch gekennzeichnet, dass** das zweite 90°-Prisma (102, 103, 202, 302, 303) um eine zu einer Mantelfläche des Prismas in etwa parallele Achse (114, 115, 214, 215, 314, 316) drehbar ist.

5. System zur Bildumkehr nach einem der Ansprüche 1 bis 4, **dadurch gekennzeichnet, dass** wenigstens zwei Spiegel (107, 208, 209, 308, 309) drehbar und wenigstens zwei Spiegel (108, 109, 208, 209, 308, 309) verschiebbar gelagert sind.

6. System zur Bildumkehr nach Anspruch 5, **dadurch gekennzeichnet, dass** das System zur Bildumkehr (500, 600) zwei zu einer symmetrischen Anordnung ineinandergefügte Porro-Prismensysteme (501, 502, 601, 602) umfasst.

7. Vorsatzmodul (750) mit einem System zur Bildumkehr (751) zum Anbau an ein Mikroskop-Hauptobjektiv, insbesondere für ein Operationsmikroskop (704),
**dadurch gekennzeichnet, dass**
das System zur Bildumkehr nach einem der Ansprüche 1 bis 6 ausgebildet ist.

8. Vorsatzmodul (750) nach Anspruch 7, **dadurch gekennzeichnet, dass**
ein einstellbares Fokus-Optiksystem (752) zur Variation der Brennweite des Mikroskop-Hauptobjektivs (704) vorgesehen ist.

9. Vorsatzmodul (750) nach Anspruch 7 oder 8, **dadurch gekennzeichnet, dass**
für den Anbau des Vorsatzmoduls (750) an das Mikroskop-Hauptobjektiv (704) das System zur Bildumkehr (751) auf der zum Mikroskop-Hauptobjektiv (704) weisenden Seite des Vorsatzmoduls (750) vorgesehen ist.

10. Vorsatzmodul (750) nach Anspruch 8, **dadurch gekennzeichnet, dass**
das System zur Bildumkehr (751) als System zur Strahlvertauschung von wenigstens zwei binokularen Beobachtungsstrahlengängen (702, 703, 708) ausgelegt ist.

11. Vorsatzmodul (750) gemäß Anspruch 10, **dadurch gekennzeichnet, dass** das System zur Bildumkehr (751) als System zur Strahlvertauschung von wenigstens vier binokularen Beobachtungsstrahlengänge (702, 703, 708) ausgelegt ist.

12. Vorsatzmodul (750) gemäß einem der Ansprüche 10 oder 11, **dadurch gekennzeichnet, dass** das Fokus-Optiksystem (752) wenigstens eine Streulinse (752b) aufweist.

13. Vorsatzmodul (750) gemäß einem der Ansprüche 9 bis 12, **dadurch gekennzeichnet, dass** das Fokus-Optiksystem (752) wenigstens eine Sammellinse (752b) aufweist, die entlang der optische Achse (814) des Fokus-Optiksystems (752) bewegbar ist.

14. Vorsatzmodul (750) gemäß Anspruch 12 oder gemäß Anspruch 13, soweit Anspruch 13 auf Anspruch 12 zurückbezogen ist, **dadurch gekennzeichnet, dass** die Streulinse (152b) entlang der optischen Achse (814) des Fokus-Optiksystems (752) bewegbar ist.

15. Vorsatzmodul (750) gemäß einem der Ansprüche 7 bis 14, **dadurch gekennzeichnet, dass** das System zur Bildumkehr (751) in den Strahlengang ein- und ausschaltbar ist.

16. Vorsatzmodul (750) gemäß Anspruch 10 oder gemäß einem der Ansprüche 11 bis 15, soweit die Ansprüche 11 bis 15 auf Anspruch 10 zurückbezogen sind, **dadurch gekennzeichnet, dass** das Fokus-Optiksystem (752) zum Ein- und Ausschalten in den Strahlengang ausgelegt ist.

17. Vorsatzmodul (750) gemäß einem der Ansprüche 7-16, **dadurch gekennzeichnet, dass** zur Erzeugung eines Zwischenbildes eines menschlichen Auges (720) das Vorsatzmodul (750) eine Ophthalmoskopierlupe (753) umfasst.

18. Vorsatzmodul (750) gemäß Anspruch 17, **dadurch gekennzeichnet, dass** die Brennweiten von Fokus-Optiksystem (752) und Ophthalmoskopierlupe (753) aufeinander abgestimmt sind.

19. Mikroskop, insbesondere Operationsmikroskop (700), mit System zur Bildumkehr nach einem der Ansprüche 1 bis 6.

20. Mikroskop, insbesondere Operationsmikroskop (700), mit Vorsatzmodul (750) nach einem der Ansprüche 7-18.

## Claims

1. System for image reversal (100, 500, 600), having
- a first mirror for deflecting a beam (104) from an incidence direction in a first direction which is transverse to the incidence direction, the first mirror being constructed as the base face of a first 90° prism (102),
- a second mirror (107) for deflecting the beam deflected at the first mirror, in a second direction, which is transverse to the first direction, the second mirror (107) being constructed as the base face of a second 90° prism (103),
- a third mirror (108) for deflecting the beam, deflected at the second mirror (107), in a third direction, which is transverse to the second direction, and
- a fourth mirror (109) for deflecting the beam, deflected at the third mirror (108), in a direction which is transverse to the third direction,
- the first, second, third and fourth mirrors each having face normals which are at an angle to one another,
**characterized in that**
- the third mirror (108) and the fourth mirror (109) form a top face of a half Porro prism (101),
- and the second 90° prism (103) can be rotated about an axis (115) running transverse to the face normal (116) of its base face in such a way that it is possible to set a tilt of a face of the second 90° prism (103) to an opposite face of the first 90° prism (102).

2. System for image reversal (200, 300), having
- a first mirror for deflecting a beam (205) from an incidence direction in a first direction which is transverse to the incidence direction, the first mirror being constructed as the base face of a first 90° prism (201, 301),
- a second mirror (208, 308) for deflecting the beam deflected at the first mirror, in a second direction, which is transverse to the first direction, the second mirror (208, 308) being constructed as the base face of a second 90° prism (202, 302),
- a third mirror (209, 309) for deflecting the beam, deflected at the second mirror (107, 208, 308), in a third direction, which is transverse to the second direction, and
- a fourth mirror (210, 310) for deflecting the beam, deflected at the third mirror (108, 209, 309), in a direction which is transverse to the third direction,
- the first, second, third and fourth mirrors (107, 208, 308, 108, 209, 309, 109, 210, 310) each having face normals (116, 216, 217, 315) which are at an angle to one another, and
- the third mirror (209, 309) being constructed as the base face of a third 90° prism (203, 303), and the fourth mirror (210, 310) being constructed as the base face of a fourth 90° prism (204, 304),
- which together form a half Porro prism,
**characterized in that**
- the second 90° prism (203, 303) can be rotated about an axis (214, 314) running transverse to the face normal (216, 315) of its base face in such a way that it is possible to set a tilt of a face of the second 90° prism (202, 302) to an opposite face of the first 90° prism (201, 301).

3. System for image reversal according to one of Claims 1 or 2, **characterized in that** at least one mirror (108, 208, 209, 308, 309) can be displaced in a direction (113, 218, 314) running transverse to the reflecting surface.

4. System for image reversal according to one of Claims 1 to 3, **characterized in that** the second 90° prism (102, 103, 202, 302, 303) can be rotated about an axis (114, 115, 214, 215, 314, 316) which is approximately parallel to a lateral surface of the prism.

5. System for image reversal according to one of Claims 1 to 4, **characterized in that** at least two mirrors (107, 208, 209, 308, 309) are rotatably mounted, and at least two mirrors (108, 109, 208, 209, 308, 309) are displaceably mounted.

6. System for image reversal according to Claim 5, **characterized in that** the system for image reversal (500, 600) comprises two Porro prism systems (501, 502, 601, 602) interlocking to form a symmetrical arrangement.

7. Front-end module (750) having a system for image reversal (751) for attaching to a microscope main objective, in particular for a surgical microscope (704), **characterized in that** the system is constructed for image reversal according to one of Claims 1 to 6.

8. Front-end module (750) according to Claim 7, **characterized in that** an adjustable focusing optical system (752) is provided for varying the focal length of the microscope main objective (704).

9. Front-end module (750) according to Claim 7 or 8, **characterized in that** the system for image reversal (751) is provided on the side of the front-end module (750) facing the microscope main objective (704) for the purpose of attaching the front-end module (750) to the microscope main objective (704).

10. Front-end module (750) according to Claim 8, **characterized in that** the system for image reversal (751) is designed as a system for the beam interchange of at least two binocular observation beam paths (702, 703, 708).

11. Front-end module (750) according to Claim 10, **characterized in that** the system for image reversal (751) is designed as a system for the beam interchange of at least four binocular observation beam paths (702, 703, 708).

12. Front-end module (750) according to one of Claims 10 or 11, **characterized in that** the focusing optical system (752) has at least one scattering lens (752b).

13. Front-end module (750) according to one of Claims 9 to 12, **characterized in that** the focusing optical system (752) has at least one positive lens (752b) which can be moved along the optical axis (814) of the focusing optical system (752).

14. Front-end module (750) according to Claim 12 or according to Claim 13, to the extent that Claim 13 refers back to Claim 12, **characterized in that** the scattering lens (752b) can be moved along the optical axis (814) of the focusing optical system (752).

15. Front-end module (750) according to one of Claims 7 to 14, **characterized in that** the system for image reversal (751) can be switched into and out of the beam path.

16. Front-end module (750) according to Claim 10 or according to one of Claims 11 to 15, to the extent that Claims 11 to 15 refer back to Claim 10, **characterized in that** the focusing optical system (752) is designed to switch into and out of the beam path.

17. Front-end module (750) according to one of Claims 7-16, **characterized in that** the front-end module (750) comprises an ophthalmoscopic magnifier lens (753) in order to generate an intermediate image of a human eye (720).

18. Front-end module (750) according to Claim 17, **characterized in that** the focal lengths of the focusing optical system (752) and ophthalmoscopic magnifier lens (753) are coordinated with one another.

19. Microscope, in particular a surgical microscope (700), having a system for image reversal according to one of Claims 1 to 6.

20. Microscope, in particular a surgical microscope (700), having a front-end module (750) according to one of Claims 7-18.

## Revendications

1. Système (100, 500, 600) de redressement d'image, qui comprend:
un premier miroir pour dévier un faisceau (104) de rayons depuis une direction d'incidence jusqu'à une première direction qui lui est perpendiculaire, le premier miroir étant configuré comme surface de base d'un premier prisme (102) à 90°,
un deuxième miroir (107) pour dévier le faisceau de rayons dévié par le premier miroir dans une deuxième direction qui est perpendiculaire à la première direction, le deuxième miroir (107) étant configuré comme surface de base d'un deuxième prisme (103) à 90°,
un troisième miroir (108) pour dévier le faisceau de rayons dévié par le deuxième miroir (107) dans une troisième direction qui est perpendiculaire à la deuxième direction et
un quatrième miroir (109) pour dévier le faisceau de rayons dévié par le troisième miroir (108) dans une direction qui est perpendiculaire à la troisième direction,
les perpendiculaires aux surfaces du premier miroir, du deuxième miroir, du troisième miroir et du quatrième miroir se coupant selon un angle,
**caractérisé en ce que**:
le troisième miroir (108) et le quatrième miroir (109) forment une surface d'un demi-prisme de Porro (101) et
le deuxième prisme (103) à 90° peut être déplacé par rotation autour d'un axe (115) qui s'étend perpendiculairement à la perpendiculaire (116) à la surface de base du prisme, de telle sorte que l'on peut régler un basculement d'une surface du deuxième prisme (103) à 90° vers une surface du premier prisme (102) à 90°, qui lui fait face.

2. Système (200, 300) de redressement de l'image, qui comprend:
un premier miroir pour dévier un faisceau (205) de rayons depuis une direction d'incidence jusqu'à une première direction qui est lui est perpendiculaire, le premier miroir étant configuré comme surface de base d'un premier prisme (201, 301) à 90°,
un deuxième miroir (208, 308) pour dévier le faisceau de rayons dévié par le premier miroir dans une deuxième direction qui est perpendiculaire à la première direction, le deuxième miroir (208, 308) étant configuré comme surface de base d'un deuxième prisme (202, 302) à 90°,
un troisième miroir (209, 309) pour dévier le faisceau de rayons dévié par le deuxième miroir (107, 208, 308) dans une troisième direction qui est perpendiculaire à la deuxième direction et
un quatrième miroir (210, 310) pour dévier le faisceau de rayons dévié par le troisième miroir (108, 209, 309) dans une direction qui est perpendiculaire à la troisième direction,
les perpendiculaires (116, 216, 217, 315) aux surfaces du premier miroir, du deuxième miroir, du troisième miroir et du quatrième miroir (107, 208, 308, 108, 209, 309, 109, 210, 310) se coupant selon un angle,
le troisième miroir (209, 309) étant configuré comme surface de base d'un troisième prisme (203, 303) à 90° et le quatrième miroir (210, 310) comme surface de base d'un quatrième prisme (204, 304) à 90°, qui forment ensemble un demi prisme de Porro,
**caractérisé en ce que**:
le deuxième prisme (203, 303) à 90° peut être déplacé en rotation autour d'un axe (214, 314) qui s'étend perpendiculairement à la perpendiculaire (216, 315) à la surface de base du prisme, de telle sorte que l'on peut régler un basculement d'une surface du deuxième prisme (202, 302) à 90° vers une surface du premier prisme (201, 301) à 90°, qui lui fait face.

3. Système de redressement d'image selon l'une quelconque des revendications 1 et 2, **caractérisé en ce qu'**au moins un miroir (108, 208, 209, 308, 309) peut être déplacé dans une direction (113, 218, 314) perpendiculaire à la surface du miroir.

4. Système de redressement d'image selon l'une quelconque des revendications 1 à 3, **caractérisé en ce que** le deuxième prisme (102, 103, 202, 302, 303) à 90° peut être déplacé à rotation autour d'un axe (114, 115, 214, 215, 314, 316) à peu près parallèle à une surface de l'enveloppe du prisme.

5. Système de redressement d'image selon l'une quelconque des revendications 1 à 4, **caractérisé en ce qu'**au moins deux miroirs (107, 208, 209, 308, 309) sont agencés de manière à pouvoir être déplacés en rotation et **en ce qu'**au moins deux miroirs (108, 109, 208, 209, 308, 309) sont agencés de façon à pouvoir être déplacés par translation.

6. Système de redressement d'image selon la revendication 5, **caractérisé en ce que** le système (500, 600) de redressement d'image comprend deux systèmes (501, 502, 601, 602) de prismes de Porro imbriqués l'un dans l'autre.

7. Module supplémentaire (750) qui comprend un système (751) de redressement d'image et qui est destiné à être monté sur un objectif principal d'un microscope, en particulier d'un microscope (704) d'opération, **caractérisé en ce que** le système de redressement d'image est configuré selon l'une quelconque des revendications 1 à 6.

8. Module supplémentaire (750) selon la revendication 7, **caractérisé en ce qu'**un système optique réglable (752) de mise au point est prévu pour faire varier la focale de l'objectif principal (704) du microscope.

9. Module supplémentaire (750) selon la revendication 7 ou la revendication 8, **caractérisé en ce que** le système (751) de redressement d'image est prévu du côté du module supplémentaire (750) qui est orienté vers l'objectif principal (704) du microscope pour monter le module supplémentaire (750) sur l'objectif principal (704) du microscope.

10. Module supplémentaire (750) selon la revendication 8, **caractérisé en ce que** le système (751) de redressement d'image est conçu comme système de permutation de rayons d'au moins deux trajectoires (702, 703, 708) de rayons d'observation.

11. Module supplémentaire (750) selon la revendication 10, **caractérisé en ce que** le système (751) de redressement d'image est conçu comme système de permutation de rayons d'au moins quatre trajectoires (702, 703, 708) de rayons d'observation.

12. Module supplémentaire (750) selon l'une quelconque des revendications 10 et 11, **caractérisé en ce que** le système optique (752) de mise au point présente au moins une lentille de diffusion (752b).

13. Module supplémentaire (750) selon l'une quelconque des revendications 9 à 12, **caractérisé en ce que** le système optique (752) de mise au point présente au moins une lentille de convergence (752b) qui peut être déplacée le long de l'axe optique (814) du système optique (752) de mise au point.

14. Module supplémentaire (750) selon la revendication 12 ou selon la revendication 13 dans la mesure où la revendication 13 fait référence à la revendication 12, **caractérisé en ce que** la lentille de diffusion (152b) peut être déplacée le long de l'axe optique (814) du système optique (752) de mise au point.

15. Module supplémentaire (750) selon l'une quelconque des revendications 7 à 14, **caractérisé en ce que** le système (751) de redressement d'image peut être activé ou arrêté dans la trajectoire des rayons.

16. Module supplémentaire (750) selon la revendication 10 ou selon l'une quelconque des revendications 11 à 15 dans la mesure où les revendications 11 à 15 font référence à la revendication 10, **caractérisé en ce que** le système optique (752) de mise au point est conçu pour être activé et arrêté dans la trajectoire des rayons.

17. Module supplémentaire (750) selon l'une quelconque des revendications 7 à 16, **caractérisé en ce que** le module supplémentaire (750) comprend une loupe ophtalmoscopique (753) pour produire une image intermédiaire d'un oeil humain (720).

18. Modulé supplémentaire (750) selon la revendication 17, **caractérisé en ce que** la focale du système optique (752) de mise au point et celle de la loupe ophtalmoscopique (753) sont réglées l'une par rapport à l'autre.

19. Microscope, en particulier microscope d'opération (700), qui comprend un système de redressement d'image selon l'une quelconque des revendications 1 à 6.

20. Microscope, en particulier microscope d'opération (700), qui comprend un module supplémentaire (750) selon l'une quelconque des revendications 7 à 18.
